# EUROPÄISCHE PATENTSCHRIFT

(11) **EP 1 992 283 B1**
(45) Veröffentlichungstag und Bekanntmachung des Hinweises auf die Patenterteilung: **17.11.2010**
(21) Anmeldenummer: 07009744.9
(22) Anmeldetag: 16.05.2007
(51) Int. Cl.: A61B 5/15

(54) **Stechsystem**
Piercing system
Système de connexion

(43) Veröffentlichungstag der Anmeldung: 19.11.2008
(73) Patentinhaber: Roche Diagnostics GmbH, 68305 Mannheim (DE); F.Hoffmann-La Roche AG, 4070 Basel (CH)
(72) Erfinder: Konya, Ahmet, 67165 Waldsee (DE); Harttig, Herbert, 67434 Neustadt (DE)
(74) Vertreter: Mommer, Niels

(56) Entgegenhaltungen:
- EP-A- 1 714 613
- WO-A-2004/060143
- WO-A-2005/107596
- DE-A1- 19 705 091
- US-A- 4 416 279
- US-B1- 6 322 575

## Beschreibung

Die Erfindung geht aus von einem Stechsystem mit den im Oberbegriff des Anspruchs 1 angegebenen Merkmalen. Ein derartiges Stechsystem ist aus der EP 1 714 613 A1 bekannt.

Aus der US 6,322,575 B1 ist ein Stechsystem bekannt, das an seinem vorderen Ende zur Einstellung der Stichtiefe ein keilförmiges Element aufweist, das quer zur Stichrichtung verschoben werden kann und einen Schlitz aufweist, durch den eine Lanzette bei einem Stich hindurch tritt.

Aus der WO 2005/107596 A ist ein Stechsystem mit einem Trägerband bekannt, das Lanzetten mit dazwischen angeordneten Testfeldern trägt. Eine Probenaufnahme erfolgt dabei mittels eines Kapillarkanals in dem Stechelement. Für einen Stich wird das Stechelement auf dem Trägerband soweit verschoben, dass dem Testelement eine Körperflüssigkeitsprobe über den Kapillarkanal des Stechelements zugeführt werden kann. Die Stichbewegung des bekannten Systems dient somit zugleich auch der Aufnahme einer Körperflüssigkeitsprobe.

Aus der DE 197 05 091 A1 ist ein Stechsystem bekannt, bei dem eine Lanzettenhalterung mit einem Führungskeil verwendet wird, der bei einem Stich eine Kippbewegung der Lanzette bewirkt. Die Lanzetten des dort beschriebenen Systems weisen Kapillarkanäle auf, so dass bei einem Stich zugleich auch eine Probenaufnahme erfolgt.

Derartige Stechsysteme werden beispielsweise von Diabetikern verwendet, die mehrmals täglich ihren Blutzuckerspiegel überprüfen müssen und dafür eine Körperflüssigkeitsprobe, in der Regel Blut oder interstitielle Flüssigkeit benötigen, die aus einer mit einem Stechsystem erzeugten Stichwunde gewonnen wird.

Einen besonders hohen Benutzerkomfort bieten Stechsysteme, bei denen zum Erzeugen einer Stichwunde und dem Aufnehmen einer Probe von einer erzeugten Stichwunde dasselbe Gerät verwendet werden kann. Durch eine automatische Probenaufnahme wird einem Benutzer die Untersuchung einer Körperflüssigkeitsprobe erleichtert, was insbesondere für Personen mit einer durch Alter oder Krankheit eingeschränkten manuellen Beweglichkeit ein wichtiger Vorteil ist. Zudem besteht bei einer automatischen Probeaufnahme eine geringere Gefahr einer Probenkontermination, die zu einer Verfälschung von Messergebnissen führen könnte.

Bei einer automatischen Probeaufnahme besteht jedoch die Gefahr, dass ein zu kleiner Teil der aus einer Stichwunde austretenden Körperflüssigkeit aufgenommen wird, der für eine zuverlässige Untersuchung nicht ausreicht. Im ungünstigsten Fall kann dies dazu führen, dass ein Benutzer nach einem gescheiterten Probengewinnungsversuch einen zweiten Stich erdulden muss. Um die Gefahr eines erfolglosen Stichs mit einer ungenügenden Probenaufnahme zu reduzieren, besteht im Allgemeinen die Möglichkeit, die Stichtiefe zu erhöhen, so dass mehr Körperflüssigkeit aus einer Stichwunde austritt. Auf diese Weise lässt sich zwar die Wahrscheinlichkeit, dass eine ausreichend große Menge Körperflüssigkeit von der Stichwunde aufgenommen wird, erhöhen, jedoch führt eine größere Stichtiefe zu mehr Schmerz.

Aufgabe der Erfindung ist es deshalb, einen Weg aufzuzeigen, wie bei einem Stechsystem die Probenaufnahme verbessert werden kann.

Diese Aufgabe wird mit einem Stechsystem mit im Anspruch 1 angegebenen Merkmalen gelöst. Vorteilhafte Weiterbildungen sind Gegenstand der Unteransprüche.

Der Antrieb des Stechsystems koppelt über ein Kopplungsteil für eine Stichbewegung mit einer Lanzette und für eine Probenaufnahmebewegung mit einer Probenaufnahmeeinrichtung. Das Kopplungsteil wird deshalb bei einer Stichbewegung und bei einer Probenaufnahmebewegung aus einer Startposition mit einer Vorschubbewegung jeweils in eine Endposition und aus der Endposition mit einer Rückführbewegung zurück in die Startposition bewegt.

Obwohl die Probenaufnahmeeinrichtung für eine Probenaufnahme möglichst präzise zu der zuvor bei einer Stichbewegung erzeugten Stichwunde geführt werden soll, wurde im Rahmen der Erfindung herausgefunden, dass identische Bewegungen des Kopplungsteils bei einer Probenaufnahmebewegung und einer Stichbewegung weder notwendig noch vorteilhaft sind. Überraschenderweise kann nämlich eine effizientere Probenaufnahme erreicht werden, wenn das Kopplungsteil bei einer Probenaufnahmebewegung eine Endposition erreicht, die von einer Endposition, die das Kopplungsteil bei einer Stichbewegung erreicht, reproduzierbar abweicht.

Dies kann dadurch erreicht werden, dass das Kopplungsteil bei einer Probenaufnahmebewegung einen größeren Hub ausführt als bei einer Stichbewegung. Auf diese Weise kann der Hub bei einer Stichbewegung an eine den Anforderungen eines Benutzers entsprechenden Stichtiefe angepasst werden und trotzdem stets eine zuverlässige Probenaufnahme bewirkt werden.

Alternativ oder zusätzlich kann für eine effizientere Probenaufnahme die Endposition, die das Kopplungsteil bei einer Probenaufnahmebewegung erreicht, insbesondere auch relativ zu einer Endposition, die das Kopplungsteil bei einer Stichbewegung erreicht, seitlich verschoben sein.

Weitere Einzelheiten und Vorteile der Erfindung werden an Ausführungsbeispielen unter Bezugnahme auf die beigefügten Zeichnungen erläutert. Gleiche und einander entsprechende Bauteile sind dabei mit übereinstimmenden Bezugszeichen gekennzeichnet. Die Merkmale der Ausführungsbeispiele können einzeln und in Kombination zum Gegenstand von Ansprüchen gemacht werden. Es zeigen:
- Figur 1: ein Ausführungsbeispiel eines erfindungsgemäßen Stechsystems;
- Figur 2: eine schematische Darstellung der Position eines Kopplungsteils mit einer Lanzette in Bezug auf ein an eine Geräteöffnung angelegtes Körperteil vor einem Stich;
- Figur 3: eine Darstellung gemäß Figur 2 beim Einstich der Lanzette in das Körperteil;
- Figur 4: eine Darstellung gemäß Figur 2 vor einer Probenaufnahme;
- Figur 5: eine Darstellung gemäß Figuren 2 bis 4 bei einer Probenaufnahme;
- Figur 6: eine schematische Darstellung eines Ausführungsbeispiels einer Bewegungssteuerung zur Erzeugung unterschiedlicher Bewegungsprofile des Kopplungsteils;
- Figur 7: das in Figur 6 dargestellte Ausführungsbeispiel bei einer Probenaufnahme;
- Figur 8: ein weiteres Ausführungsbeispiel einer Bewegungssteuerung in einem Zustand vor einem Stich;
- Figur 9: eine schematische Querschnittsdarstellung des Stechsystems vor einem Stich gemäß Figur 8;
- Figur 10: eine Darstellung gemäß Figur 8 während eines Stichs;
- Figur 11: eine Darstellung gemäß Figur 9 während eines Stichs;
- Figur 12: eine Darstellung gemäß Figur 10 unmittelbar nach einem Stich;
- Figur 13: eine Darstellung gemäß Figur 11 unmittelbar nach einem Stich;
- Figur 14: eine Darstellung gemäß Figur 8 vor einer Probenaufnahmebewegung;
- Figur 15: eine Darstellung gemäß Figur 13 vor einer Probenaufnahmebewegung;
- Figur 16: eine Darstellung gemäß Figur 14 bei einer Probenaufnahme;
- Figur 17: eine Darstellung gemäß Figur 15 bei einer Probenaufnahme;
- Figur 18: eine Darstellung gemäß Figur 16 nach einer Probenaufnahme;
- Figur 19: eine Darstellung gemäß Figur 17 nach einer Probenaufnahme;
- Figur 20: ein weiteres Ausführungsbeispiel einer Bewegungssteuerung in einer Querschnittsansicht;
- Figur 21: die Bewegungssteuerung von Figur 20 in einer Draufsicht in Stichrichtung;
- Figur 22: das Ausführungsbeispiel von Figur 20 in einer Vorderansicht;
- Figur 23: das Ausführungsbeispiel von Figur 20 in einer Rückansicht;
- Figur 24: ein weiteres Ausführungsbeispiel mit dem Kopplungsteil in einer Startposition für eine Stichbewegung;
- Figur 25: eine Darstellung gemäß Figur 24 mit dem Kopplungsteil in seiner Endposition bei einer Stichbewegung;
- Figur 26: eine Darstellung gemäß Figur 24 mit dem Kopplungsteil in seiner Startposition für eine Probenaufnahmebewegung; und
- Figur 27: eine Darstellung gemäß Figur 24 mit dem Kopplungsteil in seiner Endposition bei einer Probenaufnahmebewegung.

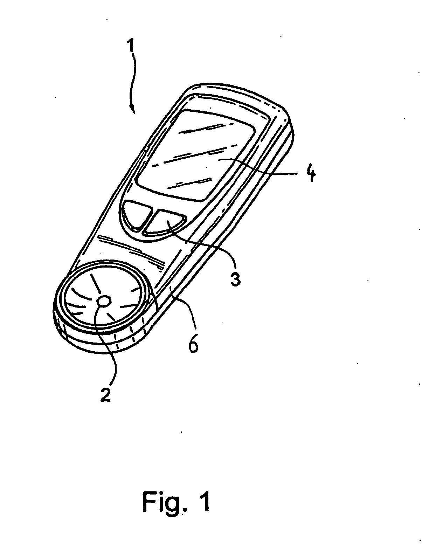

Figur 1 zeigt ein Ausführungsbeispiel eines erfindungsgemäßen Stechsystems 1 zum Gewinn einer Körperflüssigkeitsprobe. Das Stechsystem 1 hat ein Gehäuse 6 mit einer Gehäuseöffnung 2, gegen die ein Körperteil zum Erzeugen eine Stichwunde gepresst wird. Das Stechsystem 1 umfasst ferner Bedienungselemente 3 in Form von Tasten und einer Anzeigeeinrichtung 4 in Form einer Flüssigkristallanzeige zum Anzeigen von Untersuchungsergebnissen.

Bei dem dargestellten Ausführungsbeispiel umfasst das Stechsystem 1 ein mehrfach verwendbares Stechgerät, in das ein austauschbarer Lanzetten- und Testelementvorrat eingelegt werden kann. Das Stechgerät hat hierfür ein Aufnahmefach (nicht dargestellt) für einen austauschbaren Lanzetten- und Testelementvorrat. Das Aufnahmefach hat eine verschließbare Öffnung, die sich auf der Rückseite des in Figur 1 dargestellten Ausführungsbeispiels befindet. Möglich ist es aber auch, das Stechsystem als ein Stechgerät zu verwirklichen, bei dem ein Austausch des Lanzettenvorrats nicht möglich ist, so dass das Stechgerät weggeworfen wird, sobald der darin enthaltene Lanzettenvorrat aufgebraucht ist.

Als Lanzetten- und Testelementvorrat wird für das dargestellte Ausführungsbeispiel ein Trägerband verwendet, das mehrere Lanzetten trägt, zwischen denen Testelemente zur Untersuchung einer Körperflüssigkeitsprobe angeordnet sind. Die Testelemente können beispielsweise Testchemikalien aufweisen, die bei Kontakt mit einem nachzuweisenden Analyten, beispielsweise Glukose, eine Verfärbung bewirken, die photometrisch erfasst werden kann. Möglich ist beispielsweise auch eine elektrochemische Probenuntersuchung.

Das Trägeband wird mit einer Fördereinrichtung in einer Förderrichtung bewegt, so dass die Lanzetten und Testelemente nacheinander in eine Gebrauchsposition gebracht werden können. Die Fördereinrichtung kann beispielsweise eine Wickeleinrichtung sein, auf die das Förderband ähnlich wie das Band einer Tonbandkassette aufgewickelt wird, um einen Bandtransport zu bewirken. Anstelle eines Trägerbandes als Lanzetten- und Testelementvorrat können aber auch andere Formen eines Lanzetten- und/oder Testelementvorrats verwendet werden, beispielsweise Trommelmagazine.

Unter der Förderrichtung ist dabei die Richtung in Längsrichtung des Trägerbandes zu verstehen, in das Trägerband bewegt werden muss, um unbenutzte Lanzetten in die Gebrauchsposition und benutzte Lanzetten weg von der Gebrauchsposition zu bewegen.

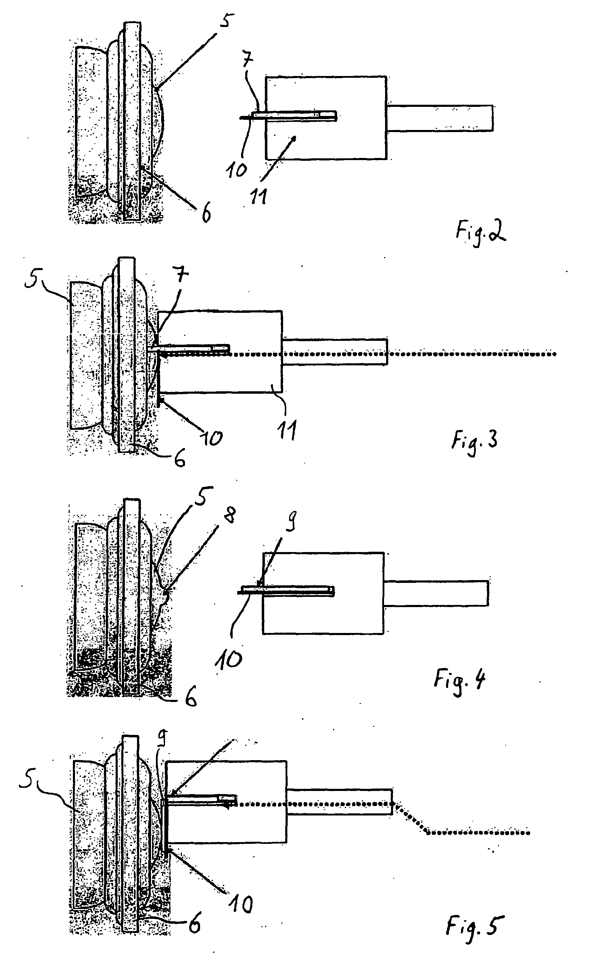

Die Figuren 2 bis 5 zeigen schematisch, wie in einem an die Gehäuseöffnung 2 angelegten Körperteil 5 mit einer Lanzette 7 eine Stichwunde erzeugt und anschließend aus der Stichwunde ausgetretene Körperflüssigkeit 8 mit einer Probenaufnahmeeinrichtung 9 aufgenommen wird. Bei der Probeaufnahmeeinrichtung 9 handelt es sich bei dem dargestellten Ausführungsbeispiel um ein Testfeld, das ebenso wie die zum Erzeugen der Stichwunde verwendete Lanzette 7 auf einem Trägerband 10 angeordnet ist.

Figur 2 zeigt ein an die Gehäuseöffnung 2 angelegtes Körperteil 5, ein Kopplungsteil 11, das eine Lanzette 7 für eine Stichbewegung und anschließend eine Probenaufnahmeeinrichtung 9 für eine Probenaufnahmebewegung an einen Antrieb koppelt. Das dargestellte Kopplungsteil 11 hat einen Spalt, durch den das Trägerband 10, das Lanzetten 7 und Testfelder 9 trägt, hindurch geführt wird. Bei dem dargestellten Ausführungsbeispiel sind die Lanzetten 7 quer zur Längsrichtung des Trägerbandes 10 angeordnet. Figur 2 zeigt eine Querschnittsansicht durch das Kopplungsteil 11 und das Trägerband 10, wobei sich eine Lanzette 7 in der Gebrauchsposition befindet, in der sie mit dem Kopplungsteil 11 für eine Stichbewegung bewegt werden kann.

Bei einer Stichbewegung wird das Kopplungsteil 11 in einer Stichrichtung bewegt, die in Figur 3 durch eine unterbrochene Linie dargestellt ist. Das Trägerband 10 wird bei der Stichbewegung in Längsrichtung gebogen, so dass sich die Spitze der Lanzette 7 von dem Trägerband 10 abhebt und in das an die Gehäuseöffnung 2 angelegte Körperteil 5 eindringt, wie es in Figur 3 dargestellt ist.

Unter Biegen in Längsrichtung ist dabei ein Biegen des Trägerbandes um eine geometrische Biegeachse zu verstehen, die sich in Längsrichtung des Bandes erstreckt. Biegen des Trägerbandes in Längsrichtung führt also dazu, dass die beiden Längsränder des Trägerbandes einen von 180° verschiedenen Winkel einschließen, beispielsweise 90° bis 135°.

Die Stichbewegung umfasst eine Vorschubbewegung, bei der das Kopplungsteil 11 aus der in Figur 2 dargestellten Startposition in die in Figur 3 dargestellte Endposition bewegt wird. In einem zweiten Teil der Stichbewegung wird das Kopplungsteil 11 mit einer Rückführbewegung in eine Startposition für eine Probenaufnahmebewegung zurückgeführt. Die Startposition für eine Probenaufnahmebewegung ist in Figur 4 gezeigt und bevorzugt mit der in Figur 2 gezeigten Startposition für eine Stichbewegung identisch.

Aus einer erzeugten Stichwunde tritt Körperflüssigkeit 8 aus, was in Figur 4 schematisch als Tropfen dargestellt ist. Zur Aufnahme von Körperflüssigkeit 8 wird eine Probenaufnahmeeinrichtung 9, bevorzugt ein Testfeld, verwendet, die bei dem dargestellten Ausführungsbeispiel ebenfalls auf dem Trägerband 10 angeordnet ist. Durch eine Förderbewegung des Trägerbandes 10 quer zur Stichrichtung wird ein Testfeld 9 in die Gebrauchsposition in dem Spalt des Kopplungsteils 11 gebracht, in der sich zuvor eine Lanzette 7 befand.

Bei einer Probenaufnahme wird das Trägerband 10 ebenso wie bei einem Stich in seiner Längsrichtung gebogen. Die Probenaufnahmebewegung ist in Figur 5 mit einer durchbrochenen Linie dargestellt. Durch Vergleich mit Figur 3 erkennt man dabei, dass sich die Probenaufnahmebewegung von einer Stichbewegung unterscheidet. Während der Vorschubbewegung einer Probenaufnahmebewegung wird das Kopplungsteil 11 seitlich abgelenkt, so dass es eine in Figur 5 dargestellte Endposition erreicht, die relativ zu der in Figur 3 dargestellten Endposition, die das Kopplungsteil bei einer Stichbewegung erreicht, seitlich verschoben ist. Diese seitliche Verschiebung quer zur Stichrichtung bewirkt, dass die Stichwunde flächig von dem in Längsrichtung gebogenen Trägerband 10 und damit auch von der auf dem Trägerband 10 angeordneten Probenaufnahmeeinrichtung 9, nämlich einem Testfeld, kontaktiert wird. Auf diese Weise kann eine Körperflüssigkeitsprobe zuverlässig aufgenommen werden.

Würde das Kopplungsteil 11 bei einer Probenaufnahmebewegung in dieselbe Endposition wie bei einer Stichbewegung gelangen, also die in Figur 3 dargestellte Endposition einnehmen, so würde das in Längsrichtung gebogene Trägerband 10 die Stichwunde nur mit seiner Biegekante berühren. Bereits die kleinste Bewegung des Körperteils 5 könnte dazu führen, dass sich die relative Position der Stichwunde zwischen Stichbewegung und Probenaufnahmebewegung geringfügig ändert und die Biegekante des Trägerbandes 10 von der Stichwunde so weit entfernt wäre, dass Körperflüssigkeit nicht oder nur in unzureichender Menge aufgenommen werden könnte. Diese Gefahr besteht bei einer in Figur 5 dargestellten Probenaufnahmebewegung nicht, da die Stichwunde flächig von dem umgebogenen Trägerband 10 kontaktiert wird und deshalb auch bei einer Positionierungenauigkeit, die sich beispielsweise durch Bewegungen des Körperteils 5 in Bezug auf das Gehäuse 6 ergeben können, zuverlässig eine Körperflüssigkeitsprobe 8 aufgenommen werden kann.

Die seitliche Ablenkung des Kopplungsteils 11 beträgt bevorzugt mehr als 1 mm, damit eventuelle Positionierungenauigkeiten der Probenaufnahmeeinrichtung 9 oder Bewegungen des Körperteils 5 möglichst keine Rolle spielen. Prinzipiell kann eine verbesserte Probenaufnahme aber auch mit einer seitlichen Ablenkung, also quer zur Stichrichtung, von weniger als 1 mm erreicht werden.

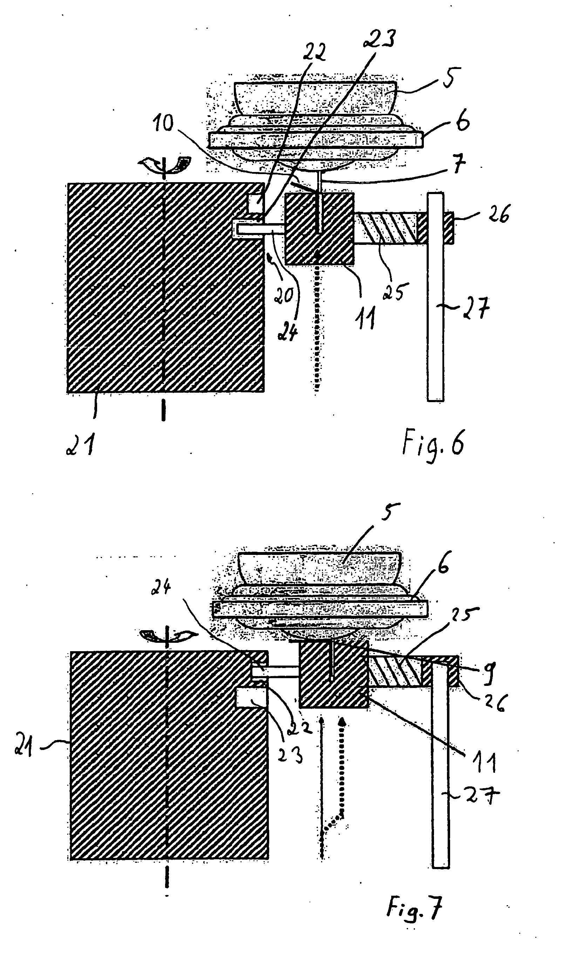

Figur 6 zeigt ein Ausführungsbeispiel einer Bewegungssteuerung 20, mit der unterschiedliche Bewegungsprofile des Kopplungsteils 11 für eine Stichbewegung und eine Probenaufnahmebewegung erreicht werden können. In Figur 6 ist das Kopplungsteil 11 zusammen mit dem Antrieb 21 bei einem Einstich in ein an die Gehäuseöffnung 2 angelegtes Körperteil 5 dargestellt. Der Antrieb 21 umfasst einen Rotor, der auf einer zylindrischen Mantelfläche zwei Nuten 22, 23 mit unterschiedlicher Tiefe aufweist. Diese beiden Nuten 22, 23 bilden jeweils eine Steuerkurve. Derartige Steuerkurven werden auch als Kulissen bezeichnet.

Das Kopplungsteil 11 hat einen Steuerkurvenreiter 24, der bei einer Stichbewegung die tiefere Nut 23 abfährt, wie es in Figur 6 dargestellt ist. Das Kopplungsteil 11 wird mit einer Feder 25 gegen den Rotor 21 gedrückt, so dass der Steuerkurvenreiter 21 stets bis zum Anschlag in die Steuerkurve 22, 23 eingreift. In der tieferen Nut 23 ist der Steuerkurvenreiter 24 von der Feder 25 unbelastet, damit bei einer Stichbewegung eine möglichst geringe Reibung auftritt. Die Feder 25 ist an einer in Stichrichtung beweglichen Führung 26 abgestützt, die bei dem dargestellten Ausführungsbeispiel ein Führungsschlitten ist, an einer sich in Stichrichtung erstreckenden Stange oder Schiene 27 beweglich gleiten kann.

Figur 7 zeigt das in Figur 6 dargestellte Ausführungsbeispiel bei einer Probenaufnahmebewegung, bei der der Steuerkurvenreiter 24 in die weniger tiefe Nut 22 eingreift. Auf diese Weise ist die in Figur 7 gezeigte Endposition des Kopplungsteils 11 bei einer Probenaufnahmebewegung im Vergleich zu seiner in Figur 6 gezeigten Endposition Stichbewegung seitlich verschoben, also quer zur Stichrichtung seitlich versetzt. Die bei einer Probenaufnahmebewegung erreichte Endposition des Kopplungsteils 11 weicht bei dem dargestellten Ausführungsbeispiel zusätzlich auch in Stichrichtung von der bei einer Stichbewegung erreichten Endposition ab. Zur Veranschaulichung sind in Figur 7 sowohl die Stichbewegung als auch die Probenaufnahmebewegung durch Pfeile angedeutet.

Bei diesem Ausführungsbeispiel führt das Kopplungsteil 11 bei einer Probenaufnahmebewegung einen größeren Hub aus als bei einer Stichbewegung, da die weniger tiefe Nut 22 in Stichrichtung oberhalb von der Nut 23 verläuft. Dies ist vorteilhaft, weil der bei einer Stichbewegung ausgeführte Hub von der eingestellten Stichtiefe abhängen kann, beispielsweise indem der Abstand der Geräteöffnung 2 von dem Antrieb 21 verstellt wird. Im Allgemeinen gilt, dass der bei einer Stichbewegung ausgeführte Hub umso kleiner ist, je kleiner die eingestellte Stichtiefe ist. Die Position der Hautoberfläche, von der mit einer Probenaufnahmebewegung eine Körperflüssigkeitsprobe 8 aufgenommen werden soll, relativ zu der Gehäuseöffnung 2 hängt jedoch nicht von der Stichtiefe ab. Indem der Hub des Kopplungsteils 11 bei einer Probenaufnahmebewegung größer als bei einer Stichbewegung ist, kann auch bei unterschiedlichen Stichtiefen stets eine zuverlässige Probenaufnahme erfolgen.

Durch die Drehrichtung des Rotors 21 wird bei dem dargestellten Ausführungsbeispiel bestimmt, welche der beiden Steuerkurven 22, 23 von dem Steuerkurvenreiter 24 abgefahren wird. Die Drehrichtung des Rotors 21 ist in den Figuren 6, 7 durch Pfeile angedeutet. Die beiden die Steuerkurven bildenden Nuten 22, 23 gehen an dem von der Gehäuseöffnung 2 abgewandten Ende des Rotors 21 ineinander über. Eine Probenaufnahmebewegung und eine Stichbewegung können bei dem dargestellten Ausführungsbeispiel als aus derselben Startposition beginnen.

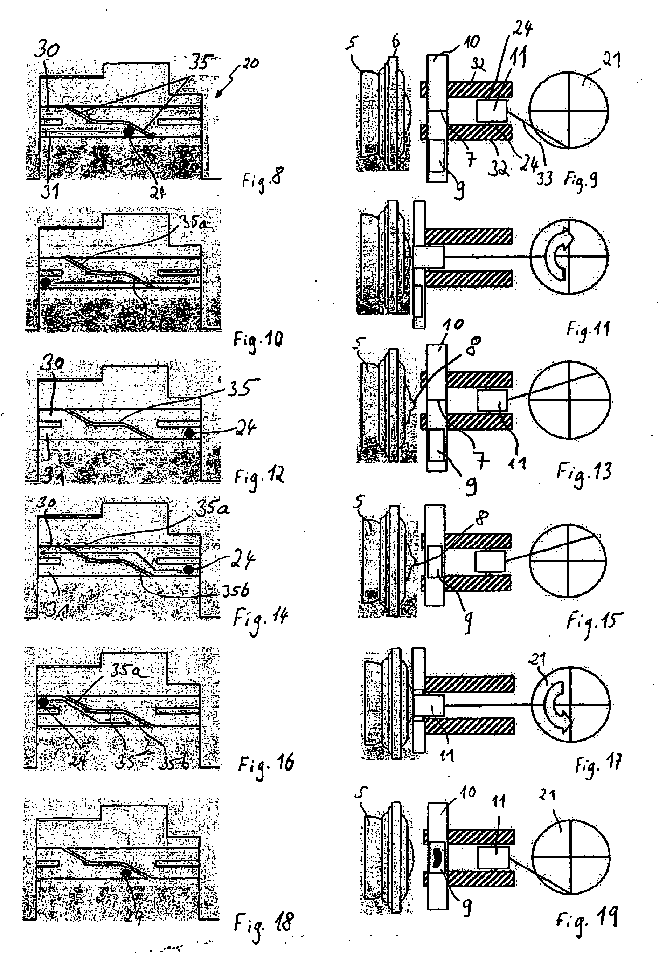

Figur 8 zeigt ein weiteres Ausführungsbeispiel einer Bewegungssteuerung 20, mit der unterschiedliche Bewegungen des Kopplungsteils 11 bei einer Stichbewegung und einer Probenaufnahmebewegung erreicht werden können. Die Bewegungssteuerung 20 des in Figur 8 dargestellten Ausführungsbeispiels nutzt ähnlich wie die Bewegungssteuerung des anhand der Figuren 6 und 7 beschriebenen Ausführungsbeispiels zwei unterschiedliche Steuerkurven 30, 31. Diese Steuerkurven 30, 31 sind aber nicht in der Mantelfläche eines Rotors angeordnet, sondern verlaufen in einem Führungselement, das sich in Stichrichtung erstreckt. Mittels einer Weiche 35 wird bestimmt, welche der Steuerkurven 30, 31 von dem Steuerkurvenreiter 24 abgefahren wird. Die Weiche 35 hat an den Seitenwänden der Nut anliegende Schenkel, die von den Seitenwänden abgehoben werden können und anschließend durch Federkraft wieder in ihre in Figur 8 gezeigte Ausgangsposition zurückkehren.

Eine schematische Darstellung eines Stechsystems mit einer Bewegungssteuerung 20 ist in Figur 9 gezeigt. Das Kopplungsteil 11 dieses Ausführungsbeispiels ist zwischen zwei Führungselementen 32 angeordnet, die jeweils Steuerkurven 30, 31 aufweisen, in die Steuerkurvenreiter 24 eingreifen. Prinzipiell genügt zwar ein einziges derartiges Führungselement 32 mit Steuerkurven 30, 31, jedoch lassen sich Kippmomente durch Verwendung von zwei Führungselementen 32 mit Steuerkurven 30, 31 reduzieren.

Das Kopplungsteil 11 wird bei dem dargestellten Stechsystem über einen Rotor 21 angetrieben, mit dem das Kopplungsteil über eine Pleuelstange 33 verbunden ist. Der Rotor 21 ist mit einem mechanischen Energiespeicher, beispielsweise einer Spiralfeder verbunden, die bei Auslösen einer Stich- oder Probenaufnahmebewegung die erforderliche Antriebskraft zur Verfügung stellt.

In Figur 9 ist das Kopplungsteil 11 in seiner Startposition vor einem Stich gezeigt. Eine Lanzette 7 des Trägerbandes 10 befindet sich in der Gebrauchsposition, so dass sie durch Auslösen des Antriebs 21 in das an die Gehäuseöffnung 2 angelegte Körperteil 5 einstechen kann. Figur 8 zeigt die entsprechende Startposition des Steuerkurvenreiters 24.

Bei einem Auslösen einer Stichbewegung bewegt sich der Steuerkurvenreiter 24 geradlinig in Richtung des in Figur 8 dargestellten Pfeils, so dass sich auch das Kopplungsteil 11 geradlinig aus seiner dargestellten Startposition in eine in Figur 11 gezeigte Endposition bewegt, aus der es mit einer Rückführbewegung wieder zurück in eine in Figur 13 gezeigte Startposition bewegt wird. Die dabei erreichte Endposition des Steuerkurvenreiters 24 ist in Figur 10 gezeigt. Bei der Rückführbewegung aus der in Figur 10 bzw. 11 gezeigten Endposition wird der Schenkel 35b der Weiche 35 von dem Steuerkurvenreiter kurzzeitig angehoben, so dass der Steuerkurvenreiter unter der Weiche hindurch in seine in Figur 12 dargestellte Startposition für eine Probenaufnahmebewegung rutschen kann.

In Figur 13 ist das Kopplungsteil nach erfolgtem Stich in seiner Startposition für eine Probenaufnahmebewegung dargestellt. Aus der erzeugten Einstichwunde austretende Körperflüssigkeit ist ebenfalls dargestellt. Die soeben für einen Stich verwendet Lanzette 7 befindet sich noch in der Gebrauchsposition. Durch eine Bewegung des Trägerbandes 10 in seiner Längsrichtung gelangt ein Testfeld 9 in die Gebrauchsposition, wie es in Figur 15 gezeigt ist.

In Figur 14 ist der Weg des Steuerkurvenreiters 24 bei einer Probenaufnahmebewegung durch einen Pfeil dargestellt. Die Weiche 35 bewirkt dabei, dass der Steuerkurvenreiter aus der in der Zeichnung unteren Steuerkurve 31 in die obere Steuerkurve 30 abgelenkt wird. Der Schenkel 35a wird bei einer Probenaufnahmebewegung kurzzeitig nach unten abgelenkt, so dass der Steuerkurvenreiter 24 in der oberen Steuerkurve 30 in seine Endposition für eine Probenaufnahme gelangen kann, die in Figur 16 dargestellt ist.

Figur 17 zeigt entsprechend zu Figur 16 das Kopplungsteil 11 in seiner Endposition, in der das Trägerband 10 in Längsrichtung gebogen ist, so dass die in Längsrichtung verlaufenden Ränder des Trägerbands 10 aneinander angenährt sind. Die Biegung des Trägerbandes 10 bewirkt, dass das Testfeld 9 flächig an dem Körperteil 5 anliegt und eine Körperflüssigkeitsprobe aufnehmen kann.

Figur 18 zeigt den Steuerkurvenreiter 24 nach der Rückführbewegung aus der in Figur 16 dargestellten Endposition. Die in Figur 18 dargestellte Startposition entspricht der in Figur 8 dargestellten Startposition. Figur 19 zeigt entsprechend das in seine Startposition zurückgekehrte Kopplungsteil 11 sowie ein Testfeld 9 mit aufgenommener Körperflüssigkeitsprobe.

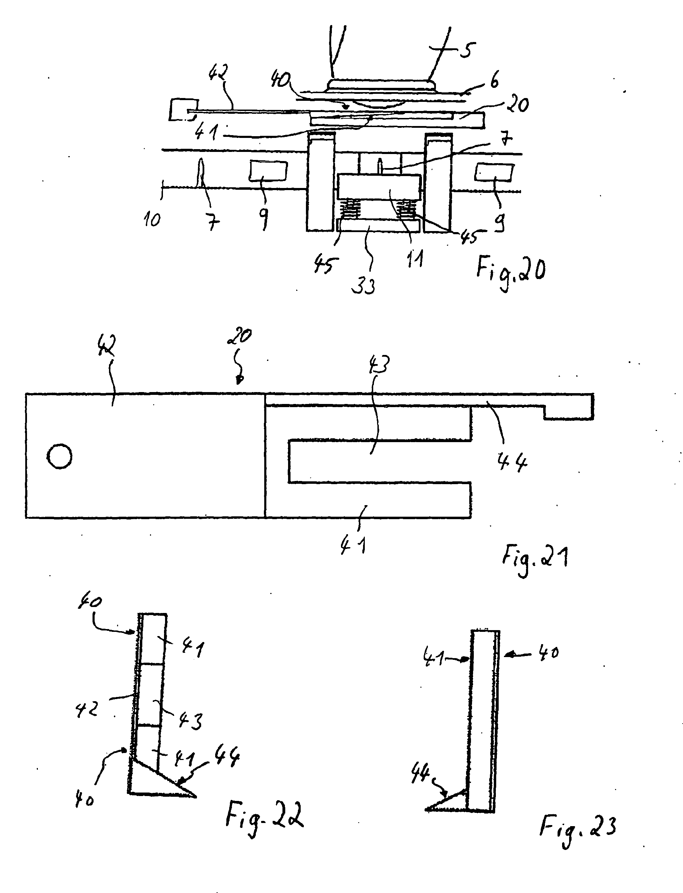

Figur 20 zeigt ein weiteres Ausführungsbeispiel einer Bewegungssteuerung 20, die bewirkt, dass das Kopplungsteil 11 bei einer Probenaufnahmebewegung eine Endposition erreicht, die relativ zu einer Endposition, die das Kopplungsteil 11 bei einer Stichbewegung erreicht, seitlich verschoben ist. Die seitliche Verschiebung ist dabei quer zur Förderrichtung des dargestellten Trägerbandes 10 und quer zur Stichrichtung.

Die Bewegungssteuerung 20 erfolgt bei dem in Figur 20 dargestellten Ausführungsbeispiel mittels eines Anschlagelements, das in Figur 21 in einer Draufsicht in Stichrichtung, in Figur 22 in einer Vorderansicht und in Figur 23 in einer Rückansicht dargestellt ist. Das Anschlagelement 20 kann quer zur Stichrichtung, bei dem dargestellten Ausführungsbeispiel in Förderrichtung des Trägerbandes 10, verstellt werden. Wie nachfolgend erläutert wird, dient das Anschlagelement 20 in der in Figur 20 dargestellten Position, die es bei einem Stich einnimmt, zur Begrenzung der Stichtiefe. Das Anschlagelement 20 hat eine der Gehäuseöffnung 2 zugewandte Unterseite 40 und eine Oberseite mit einer schräg zur Unterseite 40 verlaufenden Anschlagfläche 41, gegen die bei einem Stich das Kopplungsteil 11 anschlägt. In Stichrichtung hat das Anschlagelement 20 deshalb eine sich quer zur Stichrichtung ändernde Ausdehnung von der Anschlagfläche 41 bis zur Unterseite 40, so dass durch Bewegen des Anschlagelements 20 quer zur Stichrichtung einstellbar ist, wie weit eine Lanzette 7 bei einem Stich mit ihrer Spitze über die Unterseite 40 des Anschlagelements 20 hervorragt. Das Anschlagelement 20 ist über einen Fortsatz 42 an eine Einstelleinrichtung gekoppelt, mit der es zum Einstellen der Stichtiefe quer zur Stichrichtung, in Förderrichtung des Trägerbandes 10 verschoben werden kann.

Figur 21 zeigt, dass das Anschlagelement 20 einen Schlitz 43 aufweist. Dieser Schlitz 43 erstreckt sich durch die Anschlagfläche 41, so dass eine Lanzette 7 bei einem Stich durch den Schlitz 41 hindurchragt.

Wie insbesondere die Figuren 22 und 23 zeigen, hat das Anschlagelement 20 eine Schrägfläche 44, die sich schräg zur Stichrichtung erstreckt. Bei einer Probenaufnahmebewegung ist das Anschlagelement 20 quer zur Stichrichtung soweit verschoben, dass das Kopplungsteil 11 auf die sich in Förderrichtung erstreckende Schrägfläche 44 trifft. Die Schrägfläche bildet auf diese Weise eine Ablenkebene, an der das Kopplungsteil 11 entlang gleitet und dadurch quer zur Stichrichtung und quer zur Förderrichtung abgelenkt wird. Auf diese Weise kann ebenfalls ein seitlicher Versatz zwischen der Endposition des Kopplungsteils bei einer Probenaufnahmebewegung relativ zur Endposition des Kopplungsteils bei einer Stichbewegung bewirkt werden.

Der Antrieb des in Figur 20 dargestellten Ausführungsbeispiels kann ähnlich wie der Antrieb des in Figur 9 dargestellten Ausführungsbeispiels als Rotorantrieb ausgebildet sein, der über eine Pleuelstange an das Kopplungsteil 1 gekoppelt ist. Da durch die Anschlagfläche 41 des Anschlagteils 20 das Kopplungsteil 11 bei einer Stichbewegung vorzeitig abgestoppt werden kann, ist es über ein oder mehrere Ausgleichsfedern 45 mit der Pleuelstange 33 verbunden. Die Ausgleichsfedern 45 gleichen bei einer kleinen Einstichtiefe den von der Pleuelstange 33 bewirkten Vorschub aus, so dass der Antrieb nicht blockiert.

Die Ablenkebene 44 erstreckt sich bevorzugt wie bei dem dargestellten Ausführungsbeispiel in Förderrichtung um eine Strecke, die mindestens ebenso groß wie die Länge des Kopplungsteils 11 in Förderrichtung ist. Das Anschlagelement 20 wirkt mit einem Widerlager (nicht dargestellt) zusammen, das eine von dem Kopplungsteil 11 bei Entlanggleiten an der Ablenkebene 44 ausgeübte Kraft aufnimmt und so das Ablenkteil gegen eine Verschiebung quer zur Stichrichtung stabilisiert. Das Widerlager kann zusätzlich dazu dienen, das Anschlagelement 20 mit seiner Unterseite 40 gegen ein Körperteil 5 zu drücken, in dem eine Stichwunde erzeugt werden soll.

Die von dem Anschlagteil 20 und der mit ihm verbundenen Ablenkebene 44 gebildete Bewegungssteuerung bewirkt ebenso wie die Bewegungssteuerungen der vorstehend beschriebenen Ausführungsbeispiele eine seitliche Verschiebung der Endposition des Kopplungsteils 11 bei einer Probenaufnahmebewegung relativ zur Endposition des Kopplungsteils 11 bei einer Stichbewegung, die etwa 10-40 % der Breite des Trägerbandes 10, bevorzugt 15-35 %, insbesondere 20-30 % der Breite des Trägerbandes 10 beträgt. Bei dem in Figur 20 dargestellten Ausführungsbeispiel beträgt die seitliche Verschiebung ¼ der Breite des Trägerbandes 10. Wird das Trägerband 10 zur Probenaufnahme in seiner Mitte in Längsrichtung gefaltet, so ergibt eine derartige Verschiebung, dass das Trägerband 10 jeweils mit einem Viertel seiner Breite beidseitig über die Stichwunde hinausragt.

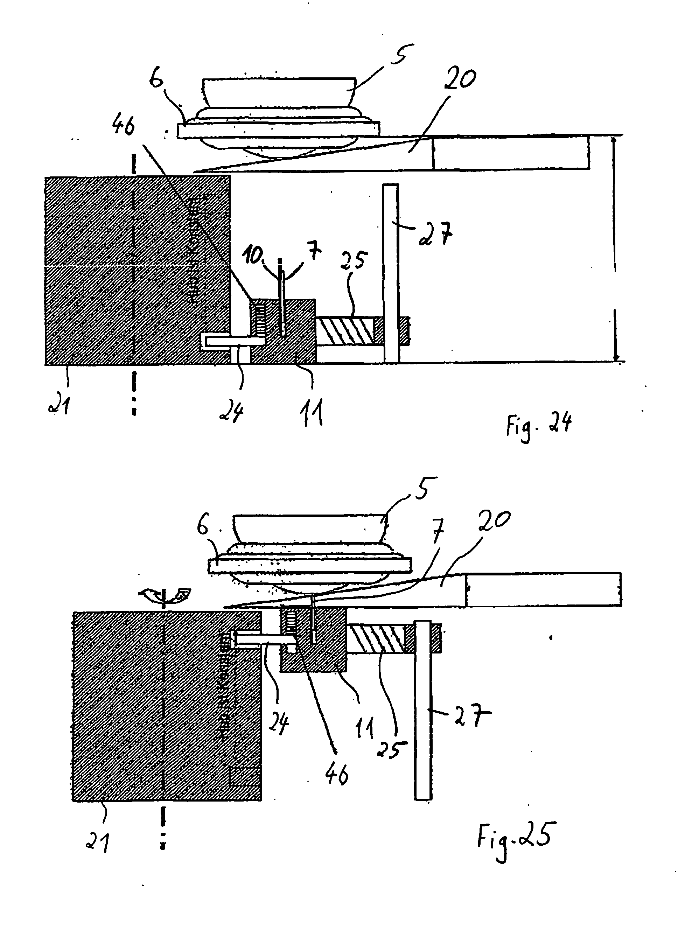

Figur 24 zeigt ein weiteres Ausführungsbeispiel eines Stechsystems, bei dem ebenso wie bei dem vorstehend beschriebenen Ausführungsbeispiel mittels eines keilförmigen Anschlagelements 20 eine Einstellung der Stichtiefe bewirkt werden kann. In Figur 24 ist das Kopplungsteil 11 in seiner Startposition für eine Stichbewegung dargestellt. Ähnlich wie bei dem anhand der Figuren 6 und 7 erläuterten Ausführungsbeispiel umfasst der Antrieb 21 einen Rotor, der auf einer zylindrischen Mantelfläche eine Nut als Steuerkurve aufweist, die von einem Steuerkurvenreiter 24 des Kopplungsteils 11 abgefahren wird.

Bei einer Stichbewegung wird das Kopplungsteil 11 durch Drehung des Rotors 21 in Stichrichtung bewegt, bis es in seiner in Figur 25 gezeigten Endposition an das Anschlagteil 20 anschlägt, das durch seine Position die Stichtiefe, mit der eine Lanzette 7 in ein an die Gehäuseöffnung angelegtes Körperteil 5 einsticht, vorgibt. Damit der Rotor 21 trotz anschlagen des Kopplungsteils 11 an das Anschlagelement 20 stets seine Drehbewegung vollenden kann, ist der Steuerkurvenreiter 24 über eine in Stichrichtung wirkende Ausgleichsfeder 46 an das Kopplungsteil 11 gekoppelt. Wenn das Kopplungsteil 11 bei einer Stichbewegung seine in Figur 25 dargestellte Endposition erreicht, indem es an das Anschlagteil 20 anschlägt, dreht sich der Rotor 21 weiter, so dass der Steuerkurvenreiter 24 weiterhin in Stichrichtung bewegt wird. Diese Bewegung wird von der Ausgleichsfeder 46 aufgenommen, die dabei komprimiert wird. Unabhängig von der Position des Anschlagteils 20 führt der Steuerkurvenreiter 24 deshalb bei einer Stichbewegung stets denselben Hub aus. Der von dem Kopplungsteil 11 ausgeführte Hub ist dagegen durch die Position des Anschlagelements 20 vorgegeben.

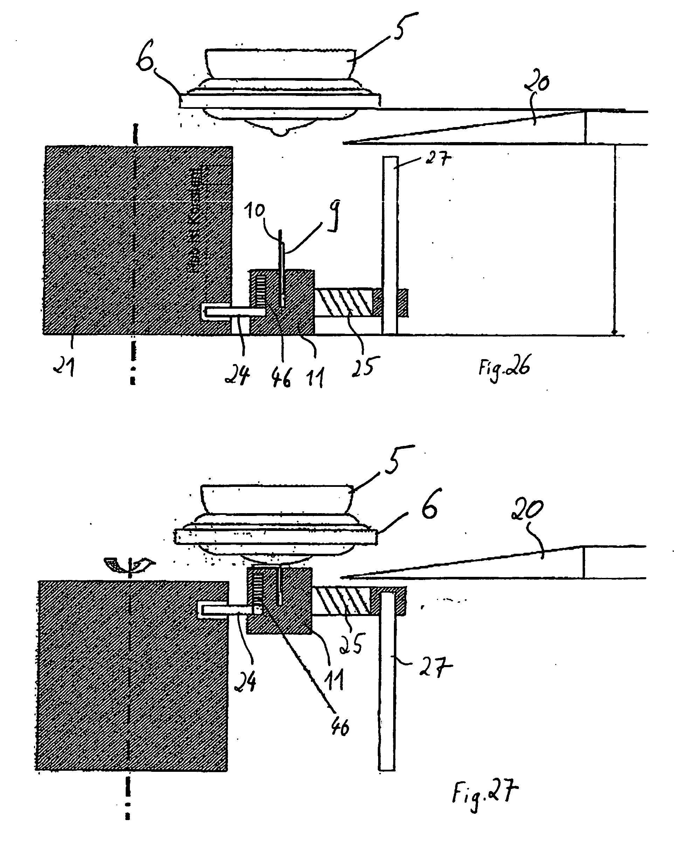

Für eine Probenaufnahme wird das Anschlagelement 20 quer zur Stichrichtung aus der Bahn des Kopplungsteils 11 wegbewegt, so dass das Kopplungsteil 11 bei einer Probenaufnahmebewegung den vollen Hub der auf der Mantelfläche des Rotors 21 angeordneten Steuerkurve ausführen kann. Figur 26 zeigt das Kopplungsteil 11 in seiner Startposition für eine Probenaufnahmebewegung. Figur 27 zeigt das Kopplungsteil 11 entsprechend in seiner Endposition bei einer Probenaufnahme.

Das Anschlagelement 20 kann zusätzlich mit einer Schrägfläche 44 versehen sein, wie sie in den Figuren 21 bis 23 dargestellt ist, um eine Ablenkung des Kopplungsteils 11 quer zur Stichrichtung bei einer Probenaufnahmebewegung zu bewirken. Zur Vereinfachung ist diese Schrägfläche 44 in den Figuren 26 und 27 nicht gezeigt. Der Rotor 21, das Kopplungsteil 11 und die Führung 27 können von einem gemeinsamen Sockel getragen werden, so dass diese Teile durch eine Schrägfläche 44 gemeinsam quer zur Stichrichtung abgelenkt werden können.

### Bezugszahlenliste

- 1: Stechsystem
- 2: Gehäuseöffnung
- 3: Bedienungselemente
- 4: Anzeigeeinrichtung
- 5: Körperteil
- 6: Gehäuse
- 7: Lanzette
- 8: Körperflüssigkeit
- 9: Probenaufnahmeeinrichtung
- 10: Trägerband
- 11: Kopplungsteil
- 20: Bewegungssteuerung
- 21: Antrieb
- 22: Steuerkurve
- 23: Steuerkurve
- 24: Steuerkurvenreiter
- 25: Feder
- 26: Führung
- 27: Schiene
- 30: Steuerkurve
- 31: Steuerkurve
- 32: Führungselement
- 33: Pleuelstange
- 35: Weiche
- 35a, b: Schenkel der Weiche
- 40: Unterseite
- 41: Anschlagseite
- 42: Fortsatz
- 43: Schlitz
- 44: Ablenkebene
- 45: Ausgleichsfeder
- 46: Ausgleichsfeder

## Patentansprüche

1. Stechsystem mit
Lanzetten (7) zum Erzeugen einer Stichwunde,
Probeaufnahmeeinrichtungen (9) zum Aufnehmen einer Körperflüssigkeitsprobe aus einer Stichwunde,
einem Gerätegehäuse (6) mit einer Gehäuseöffnung (2) zum Anlegen eines Körperteils (5), in dem eine Stichwunde erzeugt werden soll,
einem in dem Gerätegehäuse (6) angeordneten Antrieb (21), um eine Lanzette (7) zum Erzeugen einer Stichwunde und anschließend eine Probeaufnahmeeinrichtung (9) zu der erzeugten Stichwunde zu bewegen,
einem Kopplungsteil (11), um eine Lanzette (7) für eine Stichbewegung und anschließend eine Probeaufnahmeeinrichtung (9) für eine Probenaufnahmebewegung an den Antrieb (21) zu koppeln, wobei das Kopplungsteil (11) bei einer Stichbewegung und einer Probenaufnahmebewegung aus einer Startposition mit einer Vorschubbewegung jeweils in eine Endposition und aus der Endposition mit einer Rückführbewegung zurück in die Startposition bewegt wird, und
eine Bewegungssteuerung (20), die bewirkt, dass das Kopplungsteil (11) bei einer Probenaufnahmebewegung eine Endposition erreicht, die von einer Endposition, die das Kopplungsteil (11) bei einer Stichbewegung erreicht, abweicht,
**dadurch gekennzeichnet, dass** das Kopplungsteil (11) bei einer Probenaufnahmebewegung eine Endposition erreicht, die relativ zu einer Endposition, die das Kopplungsteil (11) bei einer Stichbewegung erreicht, seitlich verschoben ist.

2. Stechsystem nach Anspruch 1 **dadurch gekennzeichnet, dass** das Kopplungsteil (11) bei einer Probenaufnahmebewegung einen größeren Hub ausführt als bei einer Stichbewegung.

3. Stechsystem nach einem der vorstehenden Ansprüche, **gekennzeichnet, durch** eine Fördereinrichtung, um dem Kopplungsteil (11) nach einem Stich eine Probenaufnahmeeinrichtung (9) **durch** Bewegen der Probenaufnahmeeinrichtung (9) in einer Förderrichtung zuzuführen.

4. Stechsystem nach Anspruch 3, **dadurch gekennzeichnet, dass** das Kopplungsteil (11) bei einer Probenaufnahmebewegung eine Endposition erreicht, die relativ zu einer Endposition, die das Kopplungsteil (11) bei einer Stichbewegung erreicht, quer zur Förderrichtung verschobenen ist.

5. Stechsystem nach einem der vorstehenden Ansprüche, **dadurch gekennzeichnet, dass** die Probenaufnahmeeinrichtung (9) ein Testfeld mit Testchemikalien zur Untersuchung einer aufgenommen Körperflüssigkeitsprobe ist.

6. Stechsystem nach einem der vorstehenden Ansprüche, **dadurch gekennzeichnet, dass** die Lanzetten (7) und Probenaufnahmeeinrichtungen (9) auf einem Trägerband (10) angeordnet sind.

7. Stechsystem nach einem der vorstehenden Ansprüche, **dadurch gekennzeichnet, dass** das Trägerband (10) bei einer Stichbewegung und bei einer Probenaufnahmebewegung in Längsrichtung gebogen wird.

8. Stechsystem nach einem der vorstehenden Ansprüche, **dadurch gekennzeichnet, dass** die Bewegungssteuerung (20) eine Kurvensteuerung mit einer ersten Steuerkurve (23, 31) für eine Stichbewegung und einer zweiten Steuerkurve (24, 30) für eine Probenaufnahmebewegung umfasst.

9. Stechsystem nach einem der vorstehenden Ansprüche, **dadurch gekennzeichnet, dass** die Bewegungssteuerung (20) eine quer zur Stichrichtung verstellbare Ablenkebene (44) aufweist, die schräg zur Stichrichtung verläuft, um das Kopplungsteil (11) durch Entlanggleiten an der Ablenkebene (44) quer zur Stichrichtung abzulenken.

10. Stechsystem nach Anspruch 9, **dadurch gekennzeichnet, dass** die Ablenkebene (44) mit einem Einstellelement zum Einstellen der Stichtiefe verbunden ist, das eine Unterseite (40), die bei einem Stich dem Körperteil (5) eines Patienten zugewandt ist, und eine Oberseite mit einer Anschlagfläche (41), gegen die bei einem Stich das Kopplungselement (11) anschlägt, aufweist, wobei das Anschlagelement (20) in Stichrichtung von der Anschlagfläche (41) bis zur Unterseite (40) eine sich quer zur Stichrichtung ändernde Ausdehnung hat, so dass durch Bewegen des Anschlagelements (20) quer zur Stichrichtung einstellbar ist, wie weit eine Lanzette (7) bei einem Stich mit ihrer Spitze über die Unterseite (40) des Anschlagelements (20) hervorragt.

11. Stechsystem nach einem der vorstehenden Ansprüche, **dadurch gekennzeichnet, dass** das Kopplungsteil (11) während der Vorschubbewegung einer Probenaufnahmebewegung seitlich abgelenkt wird.

12. Stechsystem nach einem der vorstehenden Ansprüche, **dadurch gekennzeichnet, dass** das Gerätegehäuse (6) ein Aufnahmefach für einen austauschbaren Vorrat (10) von Lanzetten (7) und Probenaufnahmeeinrichtungen (9) aufweist.

## Claims

1. Pricking system comprising
lancets (7) for producing a prick wound,
sampling devices (9) for collecting a sample of a body fluid from a prick wound,
a housing (6) having an opening (2) for application of a body part (5) in which a prick wound is to be produced,
a drive (21) arranged in the housing (6) for moving a lancet (7) to produce a prick wound and then move a sampling device (9) to the prick wound so produced, and a coupling part (11) for coupling to the drive (21) a lancet (7) for a pricking movement and then a sampling device (9) for a sampling movement, the coupling part (11) being moved, during a pricking movement and a sampling movement, respectively, from a starting position into an end position by an advancing movement, and from the end position back into the starting position by a reversing movement, respectively, and
a motion control (20) which, during a sampling movement, causes the coupling part (11) to reach an end position that differs from the end position reached by the coupling part (11) during a pricking movement,
**characterized in that**
the end position reached by the coupling part (11) in a sampling movement is laterally displaced relative to an end position reached by the coupling part (11) in a pricking movement.

2. The pricking system as defined in Claim 1, **characterized in that** the coupling part (11) performs a longer travel in a sampling movement than in a pricking movement.

3. The pricking system as defined in any of the preceding claims, **characterized by** a transport facility for transporting a sampling device (9) to the coupling part (11) after a pricking operation by moving the sampling device (9) in a transport direction.

4. The pricking system as defined in Claim 3, **characterized in that** the coupling part (11) reaches an end position in a sampling movement that is displaced transversely to the transport direction relative to an end position reached by the coupling part (11) in a pricking movement.

5. The pricking system as defined in any of the preceding claims, **characterized in that** the sampling device (9) is a test field with test chemicals for testing a collected sample of a body liquid.

6. The pricking system as defined in any of the preceding claims, **characterized in that** the lancets (7) and the sampling devices (9) are arranged on a carrier tape (10).

7. The pricking system as defined in any of the preceding claims, **characterized in that** the carrier tape (10) is bent in lengthwise direction during a pricking movement and during a sampling movement.

8. The pricking system as defined in any of the preceding claims, **characterized in that** the motion control (20) comprises a cam control with a first cam (23, 31) for a pricking movement and a second cam (24, 30) for a sampling movement.

9. The pricking system as defined in any of the preceding claims, **characterized in that** the motion control (20) comprises a deflection plane (44) which can be adjusted transversely to the pricking direction and which extends obliquely to the pricking direction in order to deflect the coupling part (11) transversely to the pricking direction by sliding along the deflection plane (44).

10. The pricking system as defined in Claim 9, **characterized in that** the deflection plane (44) is connected with an adjusting element for adjusting the pricking depth, which element comprises a bottom surface (40), facing a patient's body part (5) during the pricking operation, and an upper surface with a stop surface (41) against which the coupling part (11) abuts during a pricking operation, the stop element (20) having an extension in the pricking direction, from the stop surface (41) to the bottom surface (40), that varies transversely to the pricking direction with the result that the length by which the tip of a lancet (7) will project beyond the bottom surface (40) of the stop element (20) during a pricking operation can be adjusted by moving the stop element (20) transversely to the pricking direction.

11. The pricking system as defined in any of the preceding claims, **characterized in that** the coupling part (11) is laterally deflected during the advancing step of the sampling movement.

12. The pricking system as defined in any of the preceding claims, **characterized in that** the housing (6) comprises a receptacle for an exchangeable supply (10) of lancets (7) and sampling devices (9).

## Revendications

1. Système de piqûre comprenant
des lancettes (7) pour produire une piqûre,
des dispositifs de prélèvement (9) pour prélever un échantillon de fluide corporel provenant d'une piqûre,
un boîtier d'appareil (6) pourvu d'une ouverture de boîtier (2) destinée à l'application d'une partie du corps (5) dans laquelle une piqûre doit être réalisée,
un entraînement (21) situé dans le boîtier d'appareil (6) pour déplacer une lancette (7) servant à produire une piqûre et déplacer ensuite un dispositif de prélèvement (9) vers la piqûre produite,
un élément d'accouplement (11) pour accoupler à l'entraînement (21) une lancette (7) pour un mouvement de piqûre et, ensuite, un dispositif de prélèvement (9) pour un mouvement de prélèvement, l'élément d'accouplement (11) étant déplacé, lors d'un mouvement de piqûre et d'un mouvement de prélèvement, d'une position de départ dans une position finale, à chaque fois, grâce à un mouvement d'avance et de la position finale à nouveau dans la position de départ grâce à un mouvement de retour et
une commande de mouvement (20) qui permet de faire en sorte que l'élément d'accouplement (11) atteigne, lors d'un mouvement de prélèvement, une position finale qui est différente d'une position finale atteinte par l'élément d'accouplement (11) lors d'un mouvement de piqûre, **caractérisé en ce que** l'élément d'accouplement (11) atteint, lors d'un mouvement de prélèvement, une position finale qui est décalée latéralement par rapport à une position finale atteinte par l'élément d'accouplement (11) lors d'un mouvement de piqûre.

2. Système de piqûre selon la revendication 1, **caractérisé en ce que** l'élément d'accouplement (11) exécute une plus longue course lors d'un mouvement de prélèvement que lors d'un mouvement de piqûre.

3. Système de piqûre selon l'une des revendications précédentes, **caractérisé par** un dispositif de transport pour amener vers l'élément d'accouplement (11), après une piqûre, un dispositif de prélèvement (9) amené par un déplacement du dispositif de prélèvement (9) dans une direction de transport.

4. Système de piqûre selon la revendication 3, **caractérisé en ce que** l'élément d'accouplement (11) atteint, lors d'un mouvement de prélèvement, une position finale qui est décalée transversalement à la direction de transport par rapport à une position finale atteinte par l'élément d'accouplement (11) lors d'un mouvement de piqûre.

5. Système de piqûre selon l'une des revendications précédentes, **caractérisé en ce que** le dispositif de prélèvement (9) est un élément de test contenant des substances chimiques de test pour analyser un échantillon de fluide corporel prélevé.

6. Système de piqûre selon l'une des revendications précédentes, **caractérisé en ce que** les lancettes (6) et les dispositifs de prélèvement (9) sont disposés sur une bande support (10).

7. Système de piqûre selon l'une des revendications précédentes, **caractérisé en ce que** la bande support (10) est pliée dans le sens longitudinal lors d'un mouvement de piqûre et lors d'un mouvement de prélèvement.

8. Système de piqûre selon l'une des revendications précédentes, **caractérisé en ce que** la commande de mouvement (20) comprend une commande de came avec une première came de commande (23, 31) pour un mouvement de piqûre et une seconde came de commande (24, 30) pour un mouvement de prélèvement.

9. Système de piqûre selon l'une des revendications précédentes, **caractérisé en ce que** la commande de mouvement (20) comporte un plan de déviation (44) déplaçable transversalement à la direction de piqûre, qui est incliné par rapport à la direction de piqûre, pour dévier l'élément d'accouplement (11) transversalement à la direction de piqûre par glissement le long du plan de déviation (44).

10. Système de piqûre selon la revendication 9, **caractérisé en ce que** le plan de déviation (44) est relié à un élément de réglage pour le réglage de la profondeur de piqûre qui est pourvu d'un côté inférieur (40) qui est tourné vers la partie du corps (5) d'un patient lors d'une piqûre et d'un côté supérieur présentant une surface de butée (41) contre laquelle l'élément d'accouplement (11) bute lors d'une piqûre, l'élément de butée (20) ayant une étendue dans la direction de piqûre qui se modifie transversalement à la direction de piqûre de la surface de butée (41) jusqu'au côté inférieur 40), de manière à ce que l'on puisse régler, par un déplacement de l'élément de butée (20) transversalement à la direction de piqûre, l'ampleur du dépassement de la pointe d'une lancette (7) au de-là du côté inférieur (40) de l'élément de butée (20) lors d'une piqûre.

11. Système de piqûre selon l'une des revendications précédentes, **caractérisé en ce que** l'élément d'accouplement (11) est dévié latéralement pendant le mouvement d'avance d'un mouvement de prélèvement.

12. Système de piqûre selon l'une des revendications précédentes, **caractérisé en ce que** le boîtier d'appareil (6) comprend un compartiment de réception pour une réserve remplaçable (10) de lancettes (7) et de dispositifs de prélèvement (9).
